(19) **Europäisches Patentamt**
**European Patent Office**
**Office européen des brevets**

(11) **EP 4 417 191 A1**

(12) **EUROPEAN PATENT APPLICATION**

(43) Date of publication:
**21.08.2024 Bulletin 2024/34**

(21) Application number: **23157097.9**

(22) Date of filing: **16.02.2023**

(51) International Patent Classification (IPC):
**A61K 9/00** *(2006.01)* **A61K 9/107** *(2006.01)*
**A61K 33/26** *(2006.01)* **A61K 41/00** *(2020.01)*

(52) Cooperative Patent Classification (CPC):
**A61K 41/0052; A61K 9/0009; A61K 9/1075;**
**A61K 33/26**

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**KH MA MD TN**

(71) Applicant: **Vision S.p.A.**
**20123 Milano (IT)**

(72) Inventors:
• **AGAZZI, Andrea**
**20129 Milano (IT)**
• **COLOMBO, Angelo**
**20861 Brugherio (IT)**

(74) Representative: **Palladino, Saverio Massimo et al**
**Notarbartolo & Gervasi S.p.A.**
**Viale Achille Papa, 30**
**20149 Milano (IT)**

(54) **INJECTABLE SUSPENSION OR EMULSION OF COATED MAGNETITE NANOPARTICLES FOR USE IN THE TREATMENT OF SOLID TUMORS**

(57)    The invention relates to an injectable suspension or emulsion of magnetite nanoparticles coated with an organic layer, that can be used in the treatment of solid tumors.

EP 4 417 191 A1

## Description

### FIELD OF THE INVENTION

The invention relates to an injectable suspension or emulsion of magnetite nanoparticles coated with an organic layer, which can be used in a method of treatment of solid tumors.

### STATE OF THE ART

Hyperthermia is one of the therapeutic methods proposed in oncology for the treatment of tumors. Very briefly, the principle underlying this therapeutic approach is that the heating of tumor cells in the range between about 41 and 45 °C causes their apoptosis; this treatment affects in particular cancer cells, since these are more susceptible to temperatures increases than healthy tissues ones, the latter having an effective cooling system due to their vascularization. Furthermore, the vascular system of tumors is insufficient to guarantee a correct supply of oxygen and nutrients under conditions of thermal stress. Hyperthermia can be induced with radio waves, microwaves, ultrasound waves, and other forms of energy.

Although the principle is conceptually simple, it has not been possible so far to implement it extensively in therapeutic methods or protocols due to limitations, side-effects, and practical difficulties. The main hurdle to the application of the method is the need of having a highly selective overheating of tissues, such to raise in the effective range the temperature of tumor cells without affecting the healthy surrounding tissues.

Due to these difficulties, the method has been applied only in limited cases, and only for localized treatments. A first situation where hyperthermia is used is when the tumor to be treated is near the body surface; in this case, the overheating is produced directly on the tumor from a machine outside the body. A second application of hyperthermia is by insertion of a thin, needle-like probe into the tumor for a short time, usually about 10 to 30 minutes; the tip of the probe emits a high-frequency radiation that creates very high heat and destroys the cells in the surrounding area.

For more extended areas of the body, namely, for body regions or the whole body, pure hyperthermia treatments cannot be applied, and the method is only used in support of other primary treatments, such as chemotherapy or radiotherapy; in these cases, a slight heating of the tissues is applied to kill tumor cells that are weakened by the primary treatment.

To overcome these difficulties, it has been proposed to use magnetic particles (generally nanoparticles) for reaching the tumor area and cause their heating by radiofrequency irradiation.

A great number of studies concentrated on the production and characterization of magnetic particles with controlled and reproducible characteristics; see, e.g., the review "Iron oxide based MR contrast agents: from chemistry to cell labeling", S. Laurent et al., Current Medicinal Chemistry, 2009, 16, 4712-4727; the paper "Hydrothermal preparation and characterization of ultrafine magnetite powders", Q. Yitai et al., Materials Research Bulletin, Vol. 29 (9), 1994, 953-957; the paper "Magnetic properties of bacterial magnetosomes as potential diagnostic and therapeutic tools", R. Hergt et al., Journal of Magnetism and Magnetic Materials 293 (2005) 80-86; and patent application WO 2011/073922 A1.

These studies indicate the possibility of using magnetic (nano)particles in diagnostic and therapeutic applications, but the reported characterizations are only carried out on the particles as such, or at most in vitro, without an indication of how it is possible to implement a real therapeutic hyperthermia treatment in vivo; in particular, no practical details are provided about how to convey the particles selectively in the tumor area.

Patent application WO 2015/104664 A1 discloses a method for reaching tumor areas with magnetite nanoparticles. The method consists in producing constructs comprising a plurality of magnetite nanoparticles contained in a shell of biocompatible polymers; extracting cells of the immune system from the patient; causing the cells to incorporate the constructs; and reintroducing the thus loaded cells into the body, allowing them to reach the tumor site. This method is in principle effective but does not lend itself to standardization and thus to widespread application, since it requires that the cells be extracted each time from, and then reinjected into, the specific patient.

A first object of the present invention is therefore to provide a suspension or emulsion of magnetite nanoparticles coated with an organic layer that is ready for use in hyperthermia treatments, as well as a method for using the suspension or emulsion in the treatment of solid tumors.

### SUMMARY OF INVENTION

According to the present invention, it is provided a suspension or emulsion of magnetite nanoparticles coated with an organic layer, selected among:

A) a suspension in water, added with one or more among a surfactant, a buffer system, and a stabilizer;
B) a water-based lipidic suspension or emulsion, added with one or more components selected among egg phosphatide, medium chain triglycerides, long chain triglycerides, glycerol, linoleic acid, cholesterol, and sodium oleate; and

C) a liposomal suspension or emulsion in water for injection or in an aqueous-based suspending means selected among saline, a dextrose solution, and Ringer's lactate.

**BRIEF DESCRIPTION OF THE DRAWINGS**

Figure 1 shows the evolution in time of average tumor volume for mice that received a xenograft of IGROV1-luc cells (human ovarian carcinoma cell line) and then treated according to the invention in comparison with mice not treated according to the invention.

Figure 2 shows the evolution in time of average tumor volume for mice that received a xenograft of DETROIT-562 cells (subcutaneous pharynx cancer cell line) and then treated according to the invention in comparison with mice not treated according to the invention.

**DETAILED DESCRIPTION OF THE INVENTION**

In the rest of the description, the following terms and abbreviations are used, with the meanings reported below:

- by "room temperature" it is meant a T in the range 18-30 °C;
- "NPs" stands for magnetite nanoparticles;
- for simplicity, when it is not necessary to specify further, the suspensions or emulsions of the invention will be cumulatively referred to as "injectable compositions";
- by "medium chain triglycerides" it is meant triglycerides obtained from C6-C12 fatty acids, while by "long chain triglycerides" it is meant triglycerides obtained from C14-C60 fatty acids;

In its first aspect, the present invention relates to a suspension or emulsion of NPs of magnetite ($Fe_3O_4$) coated with an organic layer as described above.

The NPs, before the coating with the organic layer, have a size between 1 and 100 nm, preferably between 10 and 60 nm, and more preferably between 20 and 30 nm. These NPs may be produced according to methods known in the art, for instance in patent application WO 2011/073922 A1. Very briefly, this method consists in: i) forming a solution of $Fe^{2+}$ ions by attack of iron metal, at a temperature between 130 and 200 °C, with an organic acid in a polyalcohol solvent, for instance glycerol; ii) oxidizing the $Fe^{2+}$ ions to $Fe^{3+}$ ions by bubbling an oxidizing gas (air) into the solution at a temperature below 100 °C and filtering off residual metallic iron; iii) treating the solution with water in a molar ratio water/$Fe^{3+}$ ions between 1.5 and 5 in the presence of a mineral acid. In this method, the size of the NPs is controlled by the molar ratio water/$Fe^{3+}$ ions, and operating in the range of molar ratios reported above ensures the obtainment of particles in the desired size range.

The NPs thus obtained are then coated with a layer of organic compounds. For the coating, the surface of the magnetite NPs is first functionalized with catechol derivatives bearing a lipophilic moiety on the phenyl ring; the two hydroxyl groups present in the catechol molecule form a polar head that bonds electrostatically to the NP surface, while the lipophilic make the assembly compatible with the polymers that form a shell around the NPs. Polymers useful for shell around the catechol-coated NPs are, among others, chitosan, sodium alginate, polymethyl methacrylate, and polysaccharides. The preferred polymer for producing the shell is a block copolymer of poly(lactic/glycolic) acid and polyethylene glycol carboxylate; for a detailed description of this copolymer, and the method for coating the NPs with the same, reference is made to patent application WO 2015/104664 A1.

The coated NPs so achieved have typically mean particle size in the range 35-60 nm, and mean polydispersity index of 0.10-0.15, indicating a narrow size distribution.

The coated NPs obtained as described above are subsequently suspended in a suspending means to prepare one of the suspensions or emulsions A), B) or C) of the invention.

These injectable compositions must of course be characterized by a set of properties to be suitable for the intended application; in particular:

- they must be sterile and pyrogen-free, in order to avoid problems in case of leaks from the tumor mass towards the surrounding healthy tissues;
- for essentially the same reason, the injectable compositions must have an osmotic concentration such to be isotonic with bodily fluids; the preparation of isotonic compositions is within the reach of the skilled person, and can be obtained for instance with a concentration of 0.9% by weight of NaCl;

- the pH of the injectable compositions must be between 7.4 and 8.8, preferably between 7.8 and 8.4;
- finally, in order to have an effective hyperthermic effect but not such to affect healthy tissues in the medium distance range from the injection site, the amount of delivered NPs must be controlled; the inventors have found that these results are obtained with a concentration of NPs in the injectable compositions in the range 10-30 mM, preferably of about 13 mM.

In a first embodiment, the injectable composition of the invention is a water-based suspension, A), and contains NPs in a concentration in the above-mentioned range. To this base composition it is added at least one further component selected among a surfactant, a buffer system, and a stabilizer.

The surfactant has the function of favoring the stability of the suspension avoiding the sedimentation of the NPs. Surfactants useful for the objects of the invention are sorbitan esters of fatty acids, commercially known as SPAN® (a family comprising SPAN® 20, sorbitol monolaurate; SPAN® 40, sorbitan monopalmitate; SPAN® 60, sorbitan monostearate; SPAN® 65, sorbitan tristearate; and other grades sold with the tradenames SPAN® 30, SPAN® 80 and SPAN® 85); polysorbate fatty acid esters commercially known as TWEEN® (available in grades 20, 21, 40, 60, 65, 80, 85); sorbitol and sorbitol esters; cyclodextrins, in particular beta-cyclodextrins; the family of sulfoalkyl ether cyclodextrin derivatives known as Captisol®; linoleic acid; and polyethylene glycol (PEG), in particular the grades commercially available as Macrogol (e.g. Macrogol 3350, Macrogol 4000, and Macrogol 6000). This component, when present, is added to suspension A) in a concentration between 1% up to 25% based upon the different excipient selected.

The buffer system has the function of maintaining the pH of the suspension in a range suitable for injection in the body, between 7.4 and 8.8 and preferably between 7.8 and 8.4; another function of the buffer, achieved through the stabilization of the pH, is to avoid the degradation of the magnetite NPs and of the organic layer coating the surface of the NPs. Buffer systems useful for keeping the pH of the suspension in this range are obtained, for instance, the phosphate buffer, obtained with the pair of salts $Na_2HPO_4/NaH_2PO_4$, the citrate buffer, obtained by mixing citric acid trisodium citrate ($C_6H_8O_7 \cdot H_2O/Na_3C_6H_5O_7 \cdot H_2O$), the histidine buffer, or some "universal buffers", so called because the pH at which they are most efficient can be regulated in a wide range depending on the ratio of components, such as the saline-sodium citrate (SSC) buffer obtained with sodium chloride and trisodium citrate and regulating the pH with hydrochloric acid, or the McIlvaine's buffer, obtained by mixing in appropriate amounts solutions of $Na_2HPO_4$ and citric acid. The preparation of these buffer solutions is well known in the field and within the reach of the average chemist.

Finally, the stabilizer of injectable composition A) is a stabilizer that can have different functions or contributions, such as:

- antioxidant, like ascorbic acid, with a concentration between 0,001 - 0,005% by weight (w/w);
- complexing, like EDTA, with a concentration between 0,025 and 0,075% w/w, cyclodextrins (Captisol® or others) with a concentration between 1 and 15% w/w, or PEG (0,1 - 10% w/w);
- preservatives, like ethyl alcohol (10-15% w/w), benzyl alcohol (0,10 - 0,15% w/w) and sodium benzoate (0,10 - 0,15% w/w)

Examples of water-based suspensions useful for the aims of the invention are:

i) a suspension comprising 3 mg of magnetite; 9 mg of NaCl; phosphate buffer in such an amount to bring the pH of the suspension in the range 8.0 ± 0.2; and water to bring the volume of the suspension to 1000 mL;
ii) a suspension comprising 3 mg of magnetite; 9 mg of NaCl; 2 mg of polyethylene glycol of average molecular weight 300 Da (commercially available as PEG Macrogol 300); phosphate buffer in such an amount to bring the pH of the suspension in the range 8.0 ± 0.2; and water to bring the volume of the suspension to 1000 mL; and
iii) a suspension comprising 3 mg of magnetite; 9 mg of NaCl; 1 mg of polysorbate 80 (polyoxyethylene (20) sorbitan monooleate, sold as TWEEN® 80); phosphate buffer in such an amount to bring the pH of the suspension in the range 8.0 ± 0.2; and water to bring the volume of the suspension to 1000 mL.

In a second embodiment, the injectable composition of the invention is a lipidic suspension or emulsion, B), in which the main component of the liquid phase is oleic acid, linoleic acid, stearic acid, polyethylene glycol to which one or more components selected among medium chain triglycerides, long chain triglycerides, glycerol, and sodium oleate can be added within a range of 0.1 up to 10% w/w.

An example of a lipidic composition of the invention is as follows:

iv) a suspension comprising 3 mg of magnetite and 1 g of linoleic acid.

Finally, in a third embodiment, the injectable composition C) of the invention is a liposomal suspension or emulsion in water for injection or in an aqueous-based suspending means selected among saline (a solution of sodium chloride in water at concentration 0.9% w/v), a dextrose solution and Ringer's lactate (a mixture of sodium chloride, sodium lactate, potassium chloride and calcium chloride in water of standard composition and very well known in the field of medicine).

Liposomes can be obtained starting from the following phospholipids: phosphatidylserine, phosphatidylcholine, phosphatidyletanolamine, phosphatidylinositol, phosphatidylethanol, phosphatidylinositol phosphate, phosphatidylglycerol.

The percentage of liposome in water is between 5 and 25% w/w.

Liposomes can be prepared following two different processes.

The first possible process starts from an organic solution as follow: adding the selected phospholipid into a dichloromethane solution and stirring to obtain a homogeneous suspension/solution. If the drug is lipophilic, adding it in the form of powder to the solution and shaking; evaporating the dichloromethane at reduced pressure by keeping the system in rotation until a film is formed, which is further dried with a more stringent vacuum system (0.2 / 0.6 microbar). Subsequently the lipid film is rehydrated with phosphate buffer. If the drug is hydrophilic, the procedure above is repeated with the only difference that the drug is added as a final step, after the lipid film is rehydrated with phosphate buffer. The mixture is then placed in a vortex mixer until it assumes a homogeneous appearance without precipitates. It is possible to proceed with a sonication (30 up to 400 Watt) if one wishes to obtain unilamellar loaded liposomes.

The second possible process is performed using a water solution. The process is as follows: adding the phospholipids to water for injection, heating the solution in a range of 40 - 65 °C and keeping it for 15 - 30 minutes. Based upon the kind of phospholipids selected, adjusting the pH between 7.5 and 9.0 or between 3.00 and 6.5, adding the NPs and stirring the solution for 30 to 120 minutes. After the stirring phase, adjusting the pH with a phosphate buffer between 7.5 and 8.5. Finally, cooling the suspension to 15 - 25 °C.

In a preferred embodiment, the injectable compositions of the invention can be provided in the form of a kit-of-parts, comprising:

- a first container containing the liquid phase of each of compositions A), B) and C), namely, water in case of composition A) oleic acid in case of composition B), and one of water for injection, saline, a water-based dextrose solution and Ringer's lactate in case of composition C);
- one or more containers containing pre-dosed amounts of lyophilized powders of superficially coated magnetite NPs and at least one component selected among surfactants, bulking agents, salts or compounds forming buffer systems, and stabilizers.

The surfactants, components of buffer systems, and stabilizers, used in preparing the kits of the invention are the same as described above with reference to composition A). The bulking agent may be selected among mannitol, lactose, sucrose, and glycine.

The provision of the injectable compositions of the invention in form of a kit has the advantage of completely avoiding aging effects of the liquid compositions, such as sedimentation of the solids or possible chemical interactions of the components. The liquid composition can then be reconstituted at the moment of use by simply mixing the contents of the various containers of the kit, generally with a gentle stirring.

The compositions of the invention are useful for treating solid tumors, in particular head and neck cancer, by injection of one of the compositions described above directly in the cancer mass followed by radiofrequency irradiation of the area of the body where said injection has occurred.

Head and neck cancer are the most indicated form of cancer to be treated with the method of the invention, since tumor masses located in these areas are relatively close to the body surface and can be easily reached with the tip of a needle for injection.

The administration of the injectable compositions of the invention occurs percutaneously via direct injection in the tumor mass through a needle; the injected amount of the compositions above is in the range of 20 μL to 1 mL, preferably between about 50 and 200 μL for a tumor volume in the range 100-500 $mm^3$. In case of tumors exceeding this volume, it is recommended to effect several injections in different points of the tumor mass, with each injection remaining in the boundaries given above.

The subsequent irradiation takes place with radiofrequencies (RF) typically in the range 10-400, with a magnetic field strength $H_0$ of between 20 and 25 kA/m, and for a time between 20 and 60 minutes, preferably of 30 $\pm$ 5 min.

Regarding the schedule of treatment, the inventors have found that the best results in terms of tumor growth inhibition are obtained with a program of 7 cycles of injection/irradiation, in which in each cycle the injection is followed by a single irradiation event, and the cycles are spaced apart 1 to 7 days.

Features and advantages of the invention will also appear more clearly from the following examples.

**INSTRUMENTS, METHODS AND EXPERIMENTAL CONDITIONS**

The tests described below were carried out, following good laboratory practice principles, on athymic nude female mice of between 5 to 7 weeks provided by Envigo RMS S.r.l. of San Pietro al Natisone, Udine (Italy). The animals were marked with indelible ink, randomized and allocated on the basis of tumor volume and clinical observations, and sacrificed at the end of the study. All the animal procedures (housing, health monitoring, restrain, dosing, ...) and ethical revision were performed according to the current Italian legislation (Legislative Decree March 4th, 2014, n. 26) enforcing the 2010/63/UE Directive on the protection of animals used for biomedical research.

RF irradiation was performed with an Ameritherm NovaStar 5W instrument of Ameritherm Inc., Scottsville (NY) USA.

MS analyses were carried out with an Applied Biosystems/Sciex triple quadrupole mass spectrometer with an Agilent Technologies 1100 HPLC pump and a CTC PAL autosampler.

**EXAMPLE 1**

This example refers to the preparation of a suspension of coated magnetite NPs.

Magnetite NPs were produced according to the polyol synthesis route described in patent application WO 2011/073922 A1; after production, the NPs were first superficially functionalized with the organic ligand N-(3,4-dihydroxyphenethyl)-dodecanamide (DDA), then dispersed in tetrahydrofuran (THF). The organic-coated magnetite nanoparticles were then encapsulated into the polymeric polylactic-co-glycolic-co-polyethylene glycol (PLGA-b-PEG-COOH) matrix mentioned in WO 2011/073922 A1. The process led to the formation of a stable phosphate buffered suspension of coated nanoparticles according to the nanoprecipitation technique using a THF:water ratio of 1:10. The so obtained suspension was dialyzed in tangential flow membranes to remove the organic solvent, the impurities, and not-reacted reagents and then concentrated to 0.3% (w/w) in $Fe_3O_4$ (150 mL).

The coated NPs in suspension were then completely characterized.

The mean particle size and mean polydispersity index for coated NPs was determined by dynamic light scattering (DLS) and found to be, respectively, 50.8 ± 0.6 nm and 0.11 ± 0.01, indicating a narrow size distribution. Transmission electron micrographs (TEM) for coated NPs showed clusters of small numbers of inorganic particles homogeneously dispersed in the polymeric matrix with an average diameter of 50 nm. $\zeta$-Potential revealed a negative surface charge, equal to -43.0 ± 0.2 mV, which can be attributed to the presence of several carboxylic acid groups on the external surface. The iron content was determined by inductively coupled plasma optical emission spectrometry and found to be equal to 2.7 mg/mL.

The suspension thus obtained was dosed in syringes for use in the subsequent biological tests.

**EXAMPLE 2**

This example refers to the ovarian cancer growth inhibition achieved with the method of the invention.

32 athymic nude female mice were subcutaneously injected on day 0 in the left flank with $5 \times 10^6$ IGROV1-luc cells (human ovarian carcinoma cell line) in 200 $\mu$l medium. The IGROVI-luc cells are commercially available from companies like Creative Biolabs, BPS Biosciences or Affimetrix.

Animals were examined daily for the appearance of tumors and for mortality and clinical signs. Toxicity was evaluated on the basis of physiological or behavioral signs of distress appeared (e.g., body weight loss ≥ 20%), paralysis, loss of body tone, tremors, bleeding from any orifice, dyspnea, hypo-activity, neurological signs and hunched posture. No unexpected deaths occurred during the tests.

Tumor progression was evaluated by caliper measurement. Tumor volume and the body weight were evaluated twice a week. The tumor volume was calculated according to formula 1 below:

$$\text{Tumor Volume (mm}^3) = \text{Length (mm)} \times \text{Width}^2 \text{ (mm}^2) \times 0.5 \qquad (1)$$

The Tumor Growth Inhibition (%T.G.I.) was calculated according to equation 2:

$$\% \text{ T.G.I.} = 100 - (\text{Mtt/Mtc} \times 100) \qquad (2)$$

wherein:

Mtt = mean tumor volume of treated group;

Mtc = mean tumor volume of control group.

When the tumors reached a mean volume of 482 mm$^3$ (day 8), mice were randomized and assigned to experimental groups, with a target of 8 mice per group.

On day 9, mice of Groups 2 to 4 received each an intratumoral single injection of 50 μL of suspension of Example 1. Mice of Group 1 (control) did not receive the injection of NPs suspension.

Radiation treatment of mice of Groups 3 and 4 started one hour after the intratumoral injection of NPs suspension. Mice were anesthetized through inhalation of isoflurane in mouse chamber with active scavenging. The radiation instrument parameters were: frequency = 360 kHz, field strength ($H_0$) = 21 kA/m.

Mice of Group 2 received no radiation, mice of Group 3 were subjected once to a 40 minutes radiation session, and mice of Group 4 were subjected to five consecutive days of 40 minutes radiation sessions, 24 hours apart from each other.

The RF radiation treatment conditions are summarized in Table 1 below:

**Table 1**

| Treatment Group | Radiation session duration (min) | Radiation session schedule |
|---|---|---|
| 1 (Control) | / | / |
| 2 | / | / |
| 3 | 40 | Once |
| 4 | 40 | 5 consecutive days, 24 hours apart |

All mice were sacrificed when reached their own humane endpoint limit: no macroscopic internal lesion was observed at the gross necroscopy.

The volume in cm$^3$ as a function of days of test for all mice in the four groups is reported in Tables 2-5 below, respectively for mice of Groups 1 to 4.

**Table 2**

| Day | Group 1 - Mice ID | | | | | | | |
|---|---|---|---|---|---|---|---|---|
| | 2 | 1 | 25 | 35 | 24 | 4 | 10 | 9 |
| 0 | 0.00 | 0.00 | 0.00 | 0.00 | 0.00 | 0.00 | 0.00 | 0.00 |
| 5 | 0.02 | 0.04 | 0.08 | 0.10 | 0.12 | 0.21 | 0.20 | 0.26 |
| 8 | 0.24 | 0.40 | 0.40 | 0.47 | 0.48 | 0.56 | 0.56 | 0.74 |
| 10 | 0.53 | 0.55 | 0.63 | 0.80 | 1.15 | 1.08 | 1.27 | 0.88 |
| 12 | 0.90 | 0.72 | 1.15 | 1.15 | 1.26 | 1.44 | 1.55 | 1.41 |
| 14 | 1.47 | 1.55 | 1.64 | 1.56 | 1.48 | 1.67 | 1.91 | 1.48 |
| 16 | 1.91 | 1.97 | 1.89 | 2.00 | 1.76 | 2.10 | 2.45 | 1.86 |
| 18 | 3.08 | 2.60 | 2.36 | 2.16 | 2.59 | 3.32 | 3.40 | 2.69 |

**Table 3**

| Day | Group 2 - Mice ID | | | | | | | |
|---|---|---|---|---|---|---|---|---|
| | 23 | 26 | 20 | 34 | 13 | 3 | 8 | 11 |
| 0 | 0.00 | 0.00 | 0.00 | 0.00 | 0.00 | 0.00 | 0.00 | 0.00 |
| 5 | 0.06 | 0.09 | 0.14 | 0.17 | 0.18 | 0.07 | 0.17 | 0.12 |
| 8 | 0.24 | 0.33 | 0.43 | 0.47 | 0.49 | 0.55 | 0.58 | 0.77 |
| 10 | 0.50 | 0.83 | 0.82 | 0.94 | 0.88 | 0.90 | 0.89 | 0.94 |
| 12 | 0.60 | 1.02 | 1.16 | 1.16 | 1.06 | 1.42 | 1.41 | 1.30 |

(continued)

| Day | Group 2 - Mice ID | | | | | | | |
|---|---|---|---|---|---|---|---|---|
| | 23 | 26 | 20 | 34 | 13 | 3 | 8 | 11 |
| 14 | 1.01 | 1.41 | 1.56 | 1.56 | 1.84 | 1.80 | 1.59 | 1.56 |
| 16 | 1.57 | 1.89 | 2.00 | 2.00 | 2.08 | 2.18 | 1.94 | 1.81 |
| 18 | 2.30 | 2.82 | 2.94 | 2.25 | 2.69 | 3.52 | 2.94 | 2.28 |

**Table 4**

| Day | Group 3 - Mice ID | | | | | | | |
|---|---|---|---|---|---|---|---|---|
| | 18 | 12 | 27 | 28 | 14 | 29 | 33 | 32 |
| 0 | 0.00 | 0.00 | 0.00 | 0.00 | 0.00 | 0.00 | 0.00 | 0.00 |
| 5 | 0.14 | 0.17 | 0.21 | 0.11 | 0.09 | 0.13 | 0.06 | 0.12 |
| 8 | 0.25 | 0.32 | 0.43 | 0.45 | 0.49 | 0.53 | 0.60 | 0.77 |
| 10 | 0.45 | 0.60 | 0.60 | 0.79 | 0.85 | 0,63 | 1.10 | 1.08 |
| 12 | 0.63 | 0.85 | 0.82 | 1.04 | 1.25 | 0.85 | 1.32 | 1.37 |
| 14 | 1.12 | 1.12 | 1.41 | 1.33 | 1.48 | 1.35 | 1.67 | 1.86 |
| 16 | 1.84 | 1.69 | 1.81 | 1.94 | 1.76 | 1.62 | 1.97 | 2.05 |
| 18 | 2.03 | 2.14 | 2.46 | 2.56 | 2.75 | 2.18 | 3.11 | 2.92 |

**Table 5**

| Day | Group 4 - Mice ID | | | | | | | |
|---|---|---|---|---|---|---|---|---|
| | 22 | 15 | 19 | 16 | 30 | 31 | 17 | 6 |
| 0 | 0.00 | 0.00 | 0.00 | 0.00 | 0.00 | 0.00 | 0.00 | 0.00 |
| 5 | 0.14 | 0.08 | 0.15 | 0.14 | 0.08 | 0.14 | 0.17 | 0.11 |
| 8 | 0.26 | 0.29 | 0.43 | 0.43 | 0.49 | 0.49 | 0.63 | 0.80 |
| 10 | 0.51 | 0.66 | 0.79 | 0.70 | 0.80 | 0.80 | 0.80 | 0.85 |
| 12 | 0.57 | 0.93 | 0.89 | 1.00 | 1.02 | 1.06 | 1.06 | 1.12 |
| 14 | 0.57 | 0.93 | 1.09 | 1.16 | 1.12 | 1.15 | 1.12 | 1.16 |
| 16 | 0.75 | 1.01 | 1.31 | 1.33 | 1.31 | 1.48 | 1.22 | 1.26 |
| 18 | 1.44 | 1.48 | 1.31 | 1.61 | 1.76 | 1.76 | 1.44 | 1.86 |
| 20 | 2.46 | 1.76 | 1.52 | 1.86 | 1.94 | 1.89 | 1.86 | 1.86 |
| 22 | - | 1.89 | 1.58 | 2.31 | 2.43 | 2.62 | 2.16 | 2.46 |
| 24 | - | 2.14 | 1.68 | - | - | - | - | - |
| 27 | - | - | 2.10 | - | - | - | - | - |

Table 6 reports the average tumor volume ($cm^3$) in each Group, along with standard error (SE) and standard deviation (SD), again as a function of days of test.

**Table 6**

| Day | Group 1 | | | Group 2 | | | Group 3 | | | Group 4 | | |
|-----|------|------|------|------|------|------|------|------|------|------|------|------|
| | AVG | SE | SD | AVG | SE | SD | AVG | SE | SD | AVG | SE | SD |
| 0 | 0.000 | 0.000 | 0.000 | 0.000 | 0.000 | 0.000 | 0.000 | 0.000 | 0.000 | 0.000 | 0.000 | 0.000 |
| 5 | 0.129 | 0.030 | 0.084 | 1.125 | 0.017 | 0.048 | 0.128 | 0.016 | 0.046 | 0.126 | 0.012 | 0.033 |
| 8 | 0.482 | 0.053 | 0.149 | 0.480 | 0.057 | 0.161 | 0.479 | 0.057 | 0.161 | 0.476 | 0.062 | 0.176 |
| 10 | 0.861 | 0.100 | 0.283 | 0.836 | 0.051 | 0.144 | 0.762 | 0.083 | 0.235 | 0.739 | 0.040 | 0.112 |
| 12 | 1.196 | 0.099 | 0.281 | 1.141 | 0.094 | 0.264 | 1.015 | 0.097 | 0.273 | 0.956 | 0.062 | 0.174 |
| 14 | 1.596 | 0.052 | 0.147 | 1.542 | 0.091 | 0.257 | 1.416 | 0.090 | 0.256 | 1.037 | 0.072 | 0.204 |
| 16 | 1.994 | 0.074 | 0.210 | 1.934 | 0.065 | 0.185 | 1.835 | 0.052 | 0.147 | 1.209 | 0.080 | 0.227 |
| 18 | 2.775 | 0.159 | 0.449 | 2.719 | 0.154 | 0.436 | 2.517 | 0.138 | 0.391 | 1.582 | 0.070 | 0.197 |
| 20 | - | - | - | - | - | - | - | - | - | 1.896 | 0.093 | 0.264 |
| 22 | - | - | - | - | - | - | - | - | - | 2.207 | 0.138 | 0.365 |
| 24 | - | - | - | - | - | - | - | - | - | 1.910 | 0.228 | 0.322 |
| 27 | - | - | - | - | - | - | - | - | - | 2.102 | - | - |

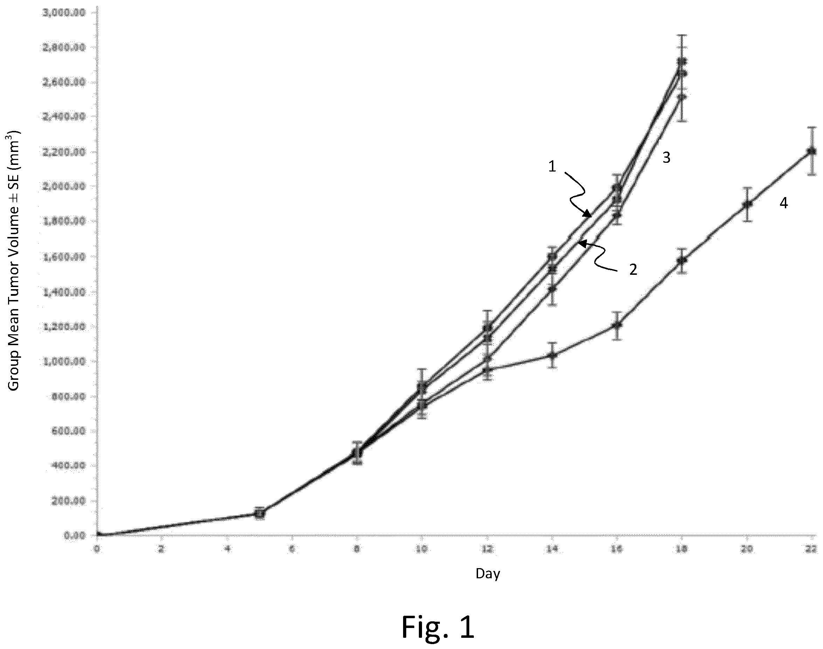

The data of average tumor volume (in mm³) growth ± SE as a function of days of test are also shown in graphical form in Fig. 1. The numbers in the figure represent the Group (1 to 4) to which the data refer; data are presented with significance level.

Table 7 reports the values of tumor growth inhibition (% T.G.I.) calculated from the data in Table 6.

**Table 7**

| Day | Group 1 | Group 2 | Group 3 | Group 4 |
|-----|---------|---------|---------|---------|
| 8 | 0.0 | 0.6 | 0.7 | 1.3 |
| 10 | 0.0 | 2.9 | 11.5 | 14.2 |
| 12 | 0.0 | 4.6 | 15.2 | 20.1 |
| 14 | 0.0 | 3.4 | 11.3 | 35.1 |
| 16 | 0.0 | 3.0 | 8.0 | 39.4 |
| 18 | 0.0 | 2.0 | 9.3 | 43.0 |

From day 15, all mice in the study showed a mild body weight loss and a mild erythema in the peritumor area. While the trend of the body weight curve loss tended to decrease around Day 15, local erythema showed no sign of recovery until the end of the study.

Control mice (Group 1) showed an exponential progression of their tumor burden.

Mice of Group 2 did not show to any significant difference with mice of Group 1.

Mice of Group 3, subjected to a single radiation session, did not show to any significant difference with mice of Group 1 either.

Mice of Group 4 showed instead a significant increase of antitumor effect, amounting to 43.0% on day 18.

No toxicity effects were observed in any of the mice of the study, confirming that the injection of coated NPs according to the invention does not lead to noxious side-effects.

## EXAMPLE 3

This example refers to the subcutaneous pharynx cancer growth inhibition achieved with the method of the invention.

49 athymic nude female mice were subcutaneously injected on day 0 in the left flank with $2 \times 10^6$ DETROIT-562 cells (subcutaneous pharynx cancer cell line) in 200 μl medium.

The same general procedure detailed in Example 2 was followed as to monitoring of the animals in the first days of cancer growth. No unexpected deaths occurred during the tests.

When the tumors reached a mean volume of 113 mm$^3$ (day 7), mice were randomized and assigned to 7 experimental groups, with a target of 7 mice per group.

Starting from day 8, mice of Groups 2 to 7 received multiple intratumoral injections of different solutions/suspensions according to two different schedules:

- four times, once a week (q7dx4); and
- seven times, every four days (q4dx7).

Specifically, Groups 2 and 3 received multiple injections of 50 μL of a buffer solution (Phosphate-buffered saline, PBS); Groups 4 to 7 received multiple injections of 50 μL of suspension of Example 1, but only the mice of Groups 6 and 7 were then anesthetized and treated with RF irradiation, which took place in the same conditions illustrated in Example 2.

Mice of Group 6 received q7dx4 NPs intratumoral treatment; every treatment was followed by three consecutive 40 minutes radiation sessions, 24 hours apart.

Mice of Group 7 received q4dx7 NPs intratumoral treatment; every treatment was followed by a single radiation session.

Mice of Group 1 (control) did not receive any injection.

The injection/irradiation conditions are summarized in Table 8 below.

**Table 8**

| Treatment Group | Test item | Injection schedule | Radiation session duration (min) | Radiation session schedule |
|---|---|---|---|---|
| 1 (Control) | / | / | / | / |
| 2 | PBS | q7dx4 | / | / |
| 3 | PBS | q4dx7 | / | / |
| 4 | NPs Ex. 1 | q7dx4 | / | / |
| 5 | NPs Ex. 1 | q4dx7 | / | / |
| 6 | NPs Ex. 1 | q7dx4 | 40 | 3 consecutive days x 4 cycles |
| 7 | NPs Ex. 1 | q4dx7 | 40 | 1 day x 7cycles |

Table 9 summarizes the average tumor volume (cm$^3$) in each Group, along with standard error (SE) and standard deviation (SD), as a function of days of test.

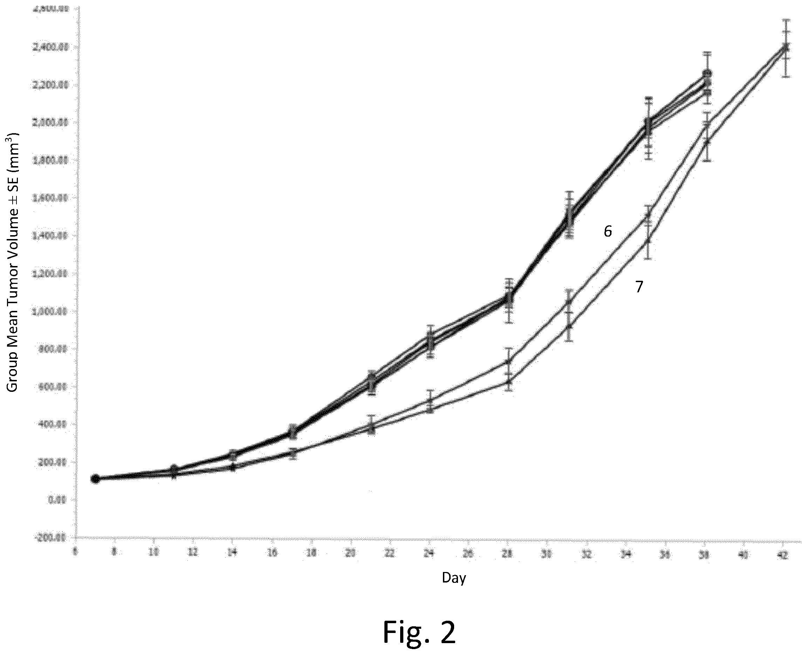

The data of average tumor volume (in mm$^3$) growth $\pm$ SE as a function of days of test are also shown in graphical form in Fig. 2. The graphs for Groups 1 to 5 are nearly superimposed in the figure, while the graphs relating to Groups 6 and 7 are identified in the figure with the corresponding number; data are presented with significance level.

**Table 9**

| Day | Group 1: Control | | | Group 2 | | | Group 3 | | | Group 4 | | | Group 5 | | | Group 6 | | | Group 7 | | |
|---|---|---|---|---|---|---|---|---|---|---|---|---|---|---|---|---|---|---|---|---|---|
| | AVG | SE | SD | AVG | SE | SD | AVG | SE | SD | AVG | SE | SD | AVG | SE | SD | AVG | SE | SD | AVG | SE | SD |
| 0 | 0.000 | 0.000 | 0.000 | 0.000 | 0.000 | 0.000 | 0.000 | 0.000 | 0.000 | 0.000 | 0.000 | 0.000 | 0.000 | 0.000 | 0.000 | 0.000 | 0.000 | 0.000 | 0.000 | 0.000 | 0.000 |
| 7 | 0.112 | 0.008 | 0.022 | 0.113 | 0.009 | 0.025 | 0.112 | 0.008 | 0.022 | 0.115 | 0.010 | 0.027 | 0.112 | 0.009 | 0.023 | 0.114 | 0.009 | 0.025 | 0.112 | 0.008 | 0.022 |
| 11 | 0.167 | 0.005 | 0.013 | 0.164 | 0.015 | 0.041 | 0.166 | 0.006 | 0.015 | 0.169 | 0.011 | 0.030 | 0.159 | 0.005 | 0.013 | 0.133 | 0.006 | 0.017 | 0.139 | 0.005 | 0.014 |
| 14 | 0.245 | 0.014 | 0.038 | 0.243 | 0.011 | 0.030 | 0.253 | 0.014 | 0.037 | 0.23 | 0.019 | 0.050 | 0.235 | 0.010 | 0.025 | 0.174 | 0.008 | 0.020 | 0.187 | 0.008 | 0.022 |
| 17 | 0.366 | 0.010 | 0.026 | 0.361 | 0.025 | 0.067 | 0.373 | 0.022 | 0.059 | 0.372 | 0.032 | 0.085 | 0.353 | 0.021 | 0.055 | 0.252 | 0.023 | 0.061 | 0.261 | 0.018 | 0.048 |
| 21 | 0.644 | 0.020 | 0.052 | 0.620 | 0.026 | 0.070 | 0.665 | 0.030 | 0.079 | 0.614 | 0.045 | 0.118 | 0.611 | 0.031 | 0.082 | 0.408 | 0.045 | 0.119 | 0.386 | 0.018 | 0.047 |
| 24 | 0.859 | 0.038 | 0.101 | 0.849 | 0.043 | 0.113 | 0.888 | 0.047 | 0.124 | 0.821 | 0.055 | 0.147 | 0.822 | 0.041 | 0.108 | 0.539 | 0.049 | 0.130 | 0.491 | 0.022 | 0.058 |
| 28 | 1.085 | 0.050 | 0.133 | 1.080 | 0.066 | 0.174 | 1.104 | 0.061 | 0.162 | 1.069 | 0.117 | 0.311 | 1.095 | 0.045 | 0.118 | 0.749 | 0.070 | 0.185 | 0.640 | 0.047 | 0.123 |
| 31 | 1.511 | 0.056 | 0.149 | 1.595 | 0.081 | 0.214 | 1.484 | 0.052 | 0.136 | 1.529 | 0.123 | 0.325 | 1.541 | 0.070 | 0.186 | 1.065 | 0.059 | 0.157 | 0.928 | 0.079 | 0.208 |
| 35 | 2.019 | 0.093 | 0.246 | 1.972 | 0.148 | 0.391 | 1.992 | 0.131 | 0.346 | 2.026 | 0.129 | 0.343 | 2.018 | 0.130 | 0.343 | 1.528 | 0.053 | 0.141 | 1.394 | 0.099 | 0.261 |
| 38 | 2.281 | 0.114 | 0.227 | 2.181 | 0.056 | 0.113 | 2.258 | 0.068 | 0.135 | 2.282 | 0.099 | 0.198 | 2.239 | 0.044 | 0.089 | 2.008 | 0.070 | 0.186 | 1.921 | 0.102 | 0.269 |
| 42 | - | - | - | - | - | - | - | - | - | - | - | - | - | - | - | 2.435 | 0.071 | 0.142 | 2.413 | 0.144 | 0.289 |

EP 4 417 191 A1

Table 10 reports the values of tumor growth inhibition (% T. G.I.) calculated from the data in Table 9.

**Table 10**

| Day | Group 1 | Group 2 | Group 3 | Group 4 | Group 5 | Group 6 | Group 7 |
|-----|---------|---------|---------|---------|---------|---------|---------|
| 7   | 0.0 | -0.6 | 0.2 | -2.3 | 0.0 | -1.1 | -0.1 |
| 11  | 0.0 | 1.5 | 0.6 | -1.8 | 4.5 | 20.4 | 16.6 |
| 14  | 0.0 | 0.7 | -3.6 | 3.7 | 3.8 | 28.8 | 23.5 |
| 17  | 0.0 | 1.5 | -2.0 | -1.5 | 3.5 | 31.2 | 28.8 |
| 21  | 0.0 | 3.7 | -3.2 | 4.7 | 5.1 | 36.6 | 40.0 |
| 24  | 0.0 | 1.2 | -3.4 | 4.4 | 4.2 | 37.2 | 42.8 |
| 28  | 0.0 | 0.5 | -1.8 | 1.5 | -0.9 | 31.0 | 41.0 |
| 31  | 0.0 | 0.4 | 1.8 | -1.2 | -2.0 | 29.5 | 38.5 |
| 35  | 0.0 | 2.3 | 1.3 | -0.3 | 0.1 | 24.3 | 31.0 |

Compared to the control group (Group 1), the treatment with PBS (Groups 2 and 3) and with the coated NPs without radiation (Groups 4 and 5) did not lead to any significant antitumor efficacy.

The intratumoral injection of coated NPs followed by multiple radiation sessions was instead able to increase the antitumor effect. A progressive delay in the tumor growth was observed until day 24, when significant maximum 37.2% ($p < 0.01$) and 42.8% ($p < 0.01$) of tumor growth inhibition were observed in Groups 6 and 7, respectively. Mann-Whitney Test revealed that Groups 6 and 7 treatment effect is significant vs Group 1 (control group). No other significant antitumor efficacy was observed in other groups.

No body weight loss and no side effects were observed.

## COMMENTS TO THE RESULTS

The data obtained in the series of tests of Examples 2 and 3 demonstrate that the method of the invention, consisting in intratumoral injection of a composition containing magnetite NPs followed by RF irradiation allows to dramatically reduce the progression of cancer growth.

One advantage of the method is that the tissues surrounding the treated cancer mass remain essentially unaffected by the treatment, so that this can be repeated several times according to needs; this is different from the known hyperthermia treatments realized with sole RF irradiation that, due to the much higher frequency ranges adopted, cause alteration of the entire area of the body and thus cannot be repeated over time.

## Claims

1. An injectable composition for percutaneous administration in a solid tumor via direct injection in the tumor mass, consisting in a suspension or emulsion of magnetite nanoparticles coated with an organic layer, selected among:

   A) a suspension in water, added with one or more among a surfactant, a buffer system, and a stabilizer;
   B) a water-based lipidic suspension or emulsion, added with one or more components selected among egg phosphatide, medium chain triglycerides, long chain triglycerides, glycerol, linoleic acid, cholesterol, and sodium oleate; and
   C) a liposomal suspension or emulsion in water for injection or in an aqueous-based suspending means selected among saline, a dextrose solution, and Ringer's lactate,

   said injectable composition having the following features:

   - it is sterile and pyrogen-free;
   - it is isotonic with bodily fluids;
   - the pH of the injectable compositions is between 7.4 and 8.8;
   - the concentration of magnetite nanoparticles in the injectable compositions in the range 10-30 mM.

2. An injectable composition according to claim 1, wherein the magnetite nanoparticles have a size between 1 and 100 nm, preferably between 10 and 60 nm, and more preferably between 20 and 30 nm.

3. An injectable composition according to claim 1 or 2, wherein the surface of the magnetite nanoparticles is first functionalized with a catechol derivative to form an assembly that is surrounded by a polymer shell, wherein the polymer is selected among chitosan, sodium alginate, polymethyl methacrylate, polysaccharides, a block copolymer of poly(lactic/glycolic) acid and polyethylene glycol carboxylate, and combinations thereof.

4. An injectable composition according to any one of the preceding claims, wherein the composition is a water-based suspension, A), comprising at least one component selected among a surfactant, a buffer system, and a stabilizer.

5. An injectable composition according to claim 4, wherein the surfactant is selected among sorbitan esters of fatty acids, polysorbate fatty acid esters, sorbitol, sorbitol esters, cyclodextrins, sulfoalkyl ether cyclodextrin derivatives, linoleic acid, polyethylene glycol, and mixtures thereof, and, when present, it is added to suspension A) in a concentration between 1% up to 25% by weight.

6. An injectable composition according to claim 4 or 5, wherein the buffer system is selected among: the phosphate buffer formed by the pair of salts $Na_2HPO_4/NaH_2PO_4$; the citrate buffer formed by citric acid trisodium citrate ($C_6H_8O_7 \cdot H_2O/Na_3C_6H_5O_7 \cdot H_2O$); the histidine buffer; the saline-sodium citrate buffer formed by sodium chloride, trisodium citrate and hydrochloric acid; and the McIlvaine's buffer, formed by solutions of $Na_2HPO_4$ and citric acid.

7. An injectable composition according to any one of claims 4 to 6, wherein the stabilizer is at least one of:

   - an antioxidant with a concentration between 0,001 - 0,005% by weight;
   - a complexing agent selected among EDTA with a concentration between 0,025 and 0,075% by weight, cyclodextrins with a concentration between 1 and 15% by weight, PEG with a concentration between 0,1 and 10% by weight, and mixtures thereof;
   - a preservative selected among ethyl alcohol with a concentration between 10 and 15% by weight, benzyl alcohol with a concentration between 0,10 and 0,15% by weight, sodium benzoate with a concentration between 0,10 and 0,15% by weight, and mixtures thereof.

8. An injectable composition according to any one of claims 1 to 3, wherein the composition is a water-based lipidic suspension or emulsion, B), and said components are added to the composition in a concentration between 0.1 and 10% by weight.

9. An injectable composition according to any one of claims 1 to 3, wherein the composition is a liposomal suspension or emulsion, B), and the liposomes containing the magnetite nanoparticles coated with an organic layer are obtained from one of the following phospholipids: phosphatidylserine, phosphatidylcholine, phosphatidyletanolamine, phosphatidylinositol, phosphatidylethanol, phosphatidylinositol phosphate, and phosphatidylglycerol.

10. An injectable composition according to claim 9, wherein the liposomes are present in a concentration between 5 and 25% by weight.

11. An injectable composition according to any one of the preceding claims in the form of a kit-of-parts, comprising:

   - a first container containing the liquid phase of any of compositions A), B) and C), namely, water in case of composition A), oleic acid in case of composition B), and one of water for injection, saline, a water-based dextrose solution and Ringer's lactate in case of composition C);
   - one or more containers containing pre-dosed amounts of lyophilized powders of superficially coated magnetite NPs and at least one component selected among surfactants, bulking agents, salts or compounds forming buffer systems, and stabilizers.

12. An injectable composition in the form of a kit-of-parts according to claim 11, wherein said bulking agent is selected among mannitol, lactose, sucrose, glycine, and mixtures thereof.

13. An injectable composition according to any one of the preceding claims for administration in an amount in the range of 20 $\mu$L to 1 mL for a tumor volume in the range 100-500 $mm^3$.

Fig. 1

Fig. 2

Europäisches
Patentamt

European
Patent Office

Office européen
des brevets

# EUROPEAN SEARCH REPORT

Application Number

EP 23 15 7097

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 2008/089478 A2 (TRITON BIOSYSTEMS INC [US]; IVKOV ROBERT [US]; FOREMAN ALLAN [US]) 24 July 2008 (2008-07-24) * paragraphs [0005], [0007], [0009]; claims 1-47 * ----- | 1-13 | INV. A61K9/00 A61K9/107 A61K33/26 A61K41/00 |
| Y | WO 2015/104664 A1 (COLOROBBIA ITALIANA SPA [IT]) 16 July 2015 (2015-07-16) * claims 1,6; figure 4 * ----- | 1-13 | |
| Y | WO 2006/125452 A1 (UNIV GENEVE [CH]; ECOLE POLYTECHNIQUE FED DE LAU [CH] ET AL.) 30 November 2006 (2006-11-30) * claims 1-22 * ----- | 1-13 | |

TECHNICAL FIELDS
SEARCHED (IPC)

A61K

The present search report has been drawn up for all claims

| Place of search | Date of completion of the search | Examiner |
|---|---|---|
| Munich | 28 July 2023 | Wörth, Christian |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
......................................................................
& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 23 15 7097

This annex lists the patent family members relating to the patent documents cited in the above-mentioned European search report.
The members are as contained in the European Patent Office EDP file on
The European Patent Office is in no way liable for these particulars which are merely given for the purpose of information.

28-07-2023

| Patent document cited in search report | | Publication date | Patent family member(s) | | Publication date |
|---|---|---|---|---|---|
| WO 2008089478 | A2 | 24-07-2008 | AU | 2008206064 A1 | 24-07-2008 |
| | | | JP | 2010516705 A | 20-05-2010 |
| | | | KR | 20100014809 A | 11-02-2010 |
| | | | SG | 177996 A1 | 28-02-2012 |
| | | | US | 2008213382 A1 | 04-09-2008 |
| | | | WO | 2008089478 A2 | 24-07-2008 |
| WO 2015104664 | A1 | 16-07-2015 | AU | 2015205350 A1 | 18-08-2016 |
| | | | BR | 112016015810 A2 | 08-08-2017 |
| | | | CA | 2935858 A1 | 16-07-2015 |
| | | | CN | 106068128 A | 02-11-2016 |
| | | | DK | 3092012 T3 | 02-09-2019 |
| | | | EP | 3092012 A1 | 16-11-2016 |
| | | | ES | 2745528 T3 | 02-03-2020 |
| | | | HU | E046306 T2 | 28-02-2020 |
| | | | JP | 6590809 B2 | 16-10-2019 |
| | | | JP | 2017501185 A | 12-01-2017 |
| | | | KR | 20160130213 A | 10-11-2016 |
| | | | MX | 367789 B | 06-09-2019 |
| | | | NZ | 722862 A | 26-08-2022 |
| | | | PL | 3092012 T3 | 31-03-2020 |
| | | | PT | 3092012 T | 18-09-2019 |
| | | | RU | 2016132482 A | 09-02-2018 |
| | | | US | 2016331850 A1 | 17-11-2016 |
| | | | US | 2021252171 A1 | 19-08-2021 |
| | | | WO | 2015104664 A1 | 16-07-2015 |
| | | | ZA | 201605425 B | 27-09-2017 |
| WO 2006125452 | A1 | 30-11-2006 | EP | 1883425 A1 | 06-02-2008 |
| | | | JP | 2008545665 A | 18-12-2008 |
| | | | US | 2009081122 A1 | 26-03-2009 |
| | | | WO | 2006125452 A1 | 30-11-2006 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2011073922 A1
- WO 2015104664 A1

**Non-patent literature cited in the description**

- **S. LAURENT et al.** Iron oxide based MR contrast agents: from chemistry to cell labeling. *Current Medicinal Chemistry,* 2009, vol. 16, 4712-4727
- **Q. YITAI et al.** Hydrothermal preparation and characterization of ultrafine magnetite powders. *Materials Research Bulletin,* 1994, vol. 29 (9), 953-957
- **R. HERGT et al.** Magnetic properties of bacterial magnetosomes as potential diagnostic and therapeutic tools. *Journal of Magnetism and Magnetic Materials,* 2005, vol. 293, 80-86